# EUROPEAN PATENT SPECIFICATION

(11) **EP 4 294 473 B1**
(45) Date of publication and mention of the grant of the patent: **10.09.2025**
(21) Application number: 22705660.3
(22) Date of filing: 04.02.2022
(51) Int. Cl.: A61L 27/46, A61L 27/50, A61L 27/56, A61L 27/58

(54) **COMPOSITIONS COMPRISING COATED CERAMIC PARTICLES AND METHODS OF MAKING THEM**
ZUSAMMENSETZUNGEN MIT BESCHICHTETEN KERAMIKPARTIKELN UND VERFAHREN ZUR HERSTELLUNG DAVON
COMPOSITIONS COMPRENANT DES PARTICULES DE CÉRAMIQUE REVÊTUES ET LEURS PROCÉDÉS DE FABRICATION

(30) Priority: 17.02.2021 US 202117177916
(43) Date of publication of application: 27.12.2023
(73) Proprietor: Warsaw Orthopedic, Inc., Memphis, TN 38132 (US)
(72) Inventor: DUNKLEY, Ian, Memphis, Tennessee 38132 (US); SELDERS, Gretchen, Memphis, Tennessee 38132 (US); JONGPAIBOONKIT, Leenaporn, Memphis, Tennessee 38132 (US)
(74) Representative: Viering, Jentschura & Partner mbB Patent- und Rechtsanwälte
(86) International application number: PCT/US2022/015198
(87) International publication number: WO 2022/177756

(56) References cited:
- US-A1- 2017 319 750
- US-A1- 2019 388 585
- US-A1- 2020 384 154
- US-A1- 2020 390 934

## Description

### BACKGROUND

Bone defects or bone voids may be caused by several different factors including, but not limited to trauma, pathological disease, or surgical intervention. Because bone provides both stability and protection to an organism, these defects or voids can be problematic. To address these defects or voids, compositions that contain both natural and synthetic materials have been developed. These compositions may, depending upon the materials contained within them, be used to repair tissues and to impart desirable biological and/or mechanical properties.

A variety of bone repair materials and bone void materials are used in the medical field. Among the known bone repair materials and bone void fillers used is autologous cancellous bone. This type of bone has the advantage of being both osteoinductive and non-immunogenic. Unfortunately, this type of bone is not frequently available. Moreover, donor site morbidity and trauma add to the limitations of autologous cancellous bone.

Allograft bone is a reasonable bone graft substitute for autologous bone. It is readily available from cadavers and avoids the surgical complications and patient morbidity associated with harvesting autologous bone. Allograft bone is essentially a load-bearing matrix comprising cross-linked collagen, hydroxyapatite, and osteoinductive bone morphogenetic proteins (BMPs). Human allograft bone is widely used in orthopedic surgery. However, allograft bone does not always have the same strength properties or the cells and proteins that can influence the growth of new bone like autograft bone provides. Further, when using allograft bone, there is a slight chance of disease transmission and a lessened effectiveness since the bone growth cells and proteins are removed during the cleansing and disinfecting process.

An alternative to autograft and allograft bone is synthetic bone material, such as ceramic based bone material. Hybrid materials composed of organic polymers coated with inorganic minerals have attracted much attention in biology and medicine due to their combination of advantageous properties. Polymeric materials are a desirable base material for biomedical applications, as they can be processed into a variety of sizes and geometries and can be designed to bioresorb in a controllable timeframe. Therefore, polymeric biomaterials have been featured in a variety of applications including medical devices, tissue engineering scaffolds, and drug delivery systems.

Calcium phosphate based mineral coatings represent desirable surfaces for biomedical applications, as they can be similar in composition to bone tissue and have been shown to promote favorable interactions with natural bone, a property termed "bioactivity". The surface modification technology associated with mineral coatings seeks to apply an apatite layer with an engineered nanoparticle sized morphology to the surface of a highly porous biphasic calcium phosphate surface. The mineral coated surface has been demonstrated to stimulate bone cells creating an enhanced cellular environment for bone healing. However, as the surface technology accomplishes bone stimulation through nanoparticle sized morphology features, the application of nanoparticle sized coatings is indistinguishable to the user and, as a result, its value proposition may not be fully realized.

Therefore, there is a need for bone implants comprising ceramic particles coated with mineral coatings that also include identification or surface markers that distinguish them from uncoated ceramic particles. Such indicia or surface markers would also increase the surface area, surface properties, hydration characteristics, and mechanical flexibility of the bone implant, allowing for improved implant-tissue biological integration.

From US 2017/319750 A1 a bone implant is known, comprising a collagen matrix, including interconnected pores, and porous calcium ceramic granules embedded within the matrix.

Further bone implant materials are known from US 2020/390934 A1, US 2020/384154 A1, and US 2019/388585 A1.

### SUMMARY

The present invention provides a bone implant with the features according to claim 1 and a method of manufacturing a bone implant with the features according to claim 9. Further preferred embodiments of the implant and the method are described in the dependent claims.

### BRIEF DESCRIPTION OF THE DRAWINGS

In part, other aspects, features, benefits, and advantages of the embodiments will be apparent regarding the following description, appended claims and accompanying drawings.
FIG. 1 is a perspective view of one embodiment of a bone implant including a plurality of lyophilized porous macroparticles coated with a mineral coating. The bone implant is coated and can have macro structures in patterns of recesses and/or projections of a variety of shapes.
FIG. 2 is a scanning electron microscope (SEM) micrograph of an uncoated MasterGraft^{®} bone implant (e.g., bone strip).
FIG. 3 is an SEM micrograph of a MasterGraft^{®} bone strip coated with a mineralized coating.
FIG. 4A and FIG. 4B are perspective views of bone implants prepared from bone strips illustrated in FIG. 2 and FIG. 3, respectively.
FIG. 5A and FIG. 5B are representations of bone implants having macro surface markers in the shape of rhomboidal design and plaid quilt design, respectively.
FIG. 6A, FIG. 6B and FIG. 7A are representations of bone implants having macro surface markers having different patterns.
FIG. 7B is a representation of a bone implant having macro surface markers in the shape of golf ball design pattern.
FIG. 8 is a bone implant having macro surface markers in the shape of a waffle.
FIG. 9 is a bone implant having macro surface markers in a corrugated shape.
FIG. 10A, FIG. 10B and FIG. 10C are bone implants having macro surface markers illustrating rectangles with triangular shapes, rectangular shaped notches, circular holes, or semi-cylindrical shapes, respectively.
FIG. 11A and FIG. 11B are representations of a bone implant having macro surface marker shaped as a rectangle strip (unfolded configuration), which can be rolled (rolled configuration) into a cake-like shape, respectively.
FIG. 12A and FIG. 12B are representations of macro surface markers illustrating circle and square designs, respectively.
FIG. 13A, FIG. 13B and FIG. 13C illustrate SEM micrographs of mesoporous surface markers on a MasterGraft^{®} bone implant strip coated with mineral coating as it appears initially and at one week after implantation at 200x magnification and 79x magnification, respectively.
FIG. 14A, FIG. 14B and FIG. 14C illustrate SEM micrographs of microporous surface markers on a MasterGraft^{®} bone implant strip. In FIG. 14A, the bone strip is mineral coated, in FIG. 14B, the SEM micrograph illustrates cell mediated resorption. FIG. 14C illustrates an SEM micrograph of an uncoated strip.
FIG. 15A is an SEM micrograph of a MasterGraft^{®} bone implant strip coated with a plate-like nanostructure of carbonate substituted, calcium-deficient hydroxyapatite (HA/TCP) shown at 1000x magnification. FIG. 15B is an SEM micrograph of the bone implant of FIG. 15A at 5000x magnification. FIG. 15C is an SEM micrograph of the bone implant of FIG. 15A at 25,000x magnification.
FIG. 16 is a perspective view of one embodiment of a bone implant including a plurality of lyophilized porous macroparticles coated with a mineral coating having macro surface markers shaped as squares.
FIG. 17 is an illustration of macro morphology of mineralized coating applied to the macro surface marker in a corrugated shape.

It is to be understood that the figures are not drawn to scale. Further, the relation between objects in a figure may not be to scale and may in fact have a reverse relationship as to size. The figures are intended to bring understanding and clarity to the structure of each object shown, and thus, some features may be exaggerated to illustrate a specific feature of a structure.

### DETAILED DESCRIPTION

### Definitions

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Similarly, when values are expressed as approximations, by use of the antecedent "about," it will be understood that the particular value forms another embodiment that is +/- 10% of the recited value. Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Treatments of the human or animal body described herein do not form part of the invention but are helpful in understanding the invention.

Notwithstanding that the numerical ranges and parameters setting forth the broad scope of this application are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

Biocompatible, as used herein, is intended to describe materials that, upon administration in vivo, do not induce undesirable long-term effects.

Bone, as used herein, refers to bone that is cortical, cancellous, or cortico-cancellous of autogenous, allogenic, xenogenic, or transgenic origin.

The term "autograft" refers to graft material harvested from the same individual patient who is also recipient of the graft, obtained surgically from non-essential donation sites in the patient.

Bone graft, as used herein, refers to any implant prepared in accordance with the embodiments described herein and therefore may include expressions such as a bone void filler.

The term "nano-sized feature" includes recesses, projections or a combination thereof that are in nanometer size.

The term "osteoinductive," as used herein, refers to the quality of being able to recruit cells from the host that have the potential to stimulate new bone formation. Any material that can induce the formation of ectopic bone in the soft tissue of an animal is considered osteoinductive.

The term "osteoinduction" refers to the ability to stimulate the proliferation and differentiation of pluripotent mesenchymal stem cells (MSCs). In endochondral bone formation, stem cells differentiate into chondroblasts and chondrocytes, laying down a cartilaginous ECM, which subsequently calcifies and is remodeled into lamellar bone. In intramembranous bone formation, the stem cells differentiate directly into osteoblasts, which form bone through direct mechanisms. Osteoinduction can be stimulated by osteogenic growth factors, although some ECM proteins can also drive progenitor cells toward the osteogenic phenotype.

The term "osteogenic" refers to the ability of a graft material to produce bone independently. To have direct osteogenic activity, the graft must contain cellular components that directly induce bone formation. For example, an allograft seeded with activated MSCs would have the potential to induce bone formation directly, without recruitment and activation of host MSC populations. Because many osteoconductive allografts also can bind and deliver bioactive molecules, their osteoinductive potential will be greatly enhanced.

The term "patient" refers to a biological system to which a treatment can be administered. A biological system can include, for example, an individual cell, a set of cells (e.g., a cell culture), an organ, or a tissue. Additionally, the term "patient" can refer to animals including, without limitation, humans.

The term "implantable" as utilized herein refers to a biocompatible device (e.g., the composition) retaining potential for successful placement within a mammal. The expression "implantable composition" and expressions of the like as utilized herein refer to an object implantable through surgery, injection, or other suitable means whose primary function is achieved either through its physical presence or mechanical properties. An example of the implantable device is the composition.

The term "moldable" includes that the composition can be shaped by hand or machine or injected into the target tissue site (e.g., bone defect, fracture, or void) into a wide variety of configurations to fit within the bone defect.

The term "cohesive" as used herein means that the composition tends to remain a singular, connected mass upon the addition of fluid, autograft bone or during manipulation, including the exhibition of the ability to be molded or shaped without breaking upon manipulating, or disintegrating or becoming unstable.

The terms "macroparticle" or "macroform" include bone material that is visible to the naked eye. The bone material can be natural bone, synthetic bone material (e.g., demineralized bone, ceramic, etc.) or a combination thereof that is solid or semi-solid before hydration. Typically, the macroparticle can be from 0.01 mm to about 50 mm in length. It is to be understood that the terms macroparticle and macroform can be used interchangeably.

The term "flowable" includes that the composition can be administered in an injectable state via a syringe and/or cannula. The composition is flowable when its consistency is fluid-like and has a viscosity that is lower than that of the viscosity of the composition when in a putty or paste form. Flowable compositions include liquid or fluid (e.g., solution, suspension, or the like) or semi-solid compositions (e.g., gels, cements) that are easy to manipulate and may be brushed, sprayed, dripped, injected, shaped and/or molded at or near the target tissue site. "Flowable" includes compositions with a low viscosity or water-like consistency to those with a high viscosity, such as a paste-like material. In various embodiments, the flowability of the composition allows it to conform to irregularities, crevices, cracks, and/or voids in the bone defect site (e.g., bone void). For example, in various embodiments, the composition may be used to fill one or more voids in an osteolytic lesion.

The term "hydrate," "hydration," "hydratable," "hydrating" or "hydrated" refers to adding an amount of fluid to a composition to increase the amount of moisture content in the composition to form a putty or paste that is flowable.

The term "dehydrated" or "dehydration" refers to a composition that contains a small amount of residual moisture or no moisture content and can be in the form of a dry composition. The dehydrated composition can have a moisture content from about 0 to about 10% based on the total weight of the composition. In some embodiments, when a composition is dehydrated, fluid can be added to the composition to hydrate the composition. A dehydrated composition includes a lyophilized or freeze-dried composition.

The term "bone marrow aspirate" or "BMA" refers to the withdrawal of bone marrow fluid through a syringe and needle to harvest the bone marrow fluid from the patient. Bone marrow aspirate comprises fluid that contains a heterogeneous mix of stem and progenitor cells, platelets, and white blood cells. The bone marrow aspirate can be harvested from various sources in the body including, but not limited to, the iliac crest.

The term "soluble collagen" refers to the solubility of individual tropocollagen molecules in acidic aqueous environments. Tropocollagen may be considered the monomeric unit of collagen fibers and its triple helix structure is well recognized.

"Insoluble collagen" as used herein refers to collagen that cannot be dissolved in an aqueous alkaline or in any inorganic salt solution without chemical modification, and includes for example hides, splits and other mammalian or reptilian coverings. For example, "natural insoluble collagen" can be derived from the corium, which is the intermediate layer of an animal hide (e. g. bovine, porcine, fish, etc.) that is situated between the grain and the flesh sides.

### Bone Implants

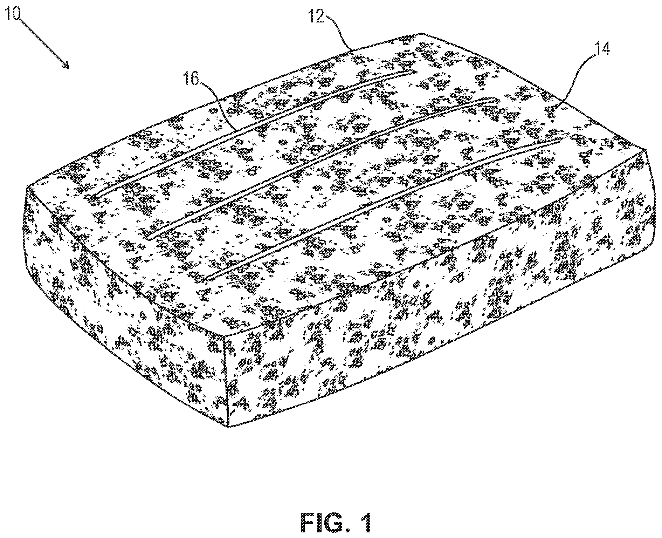

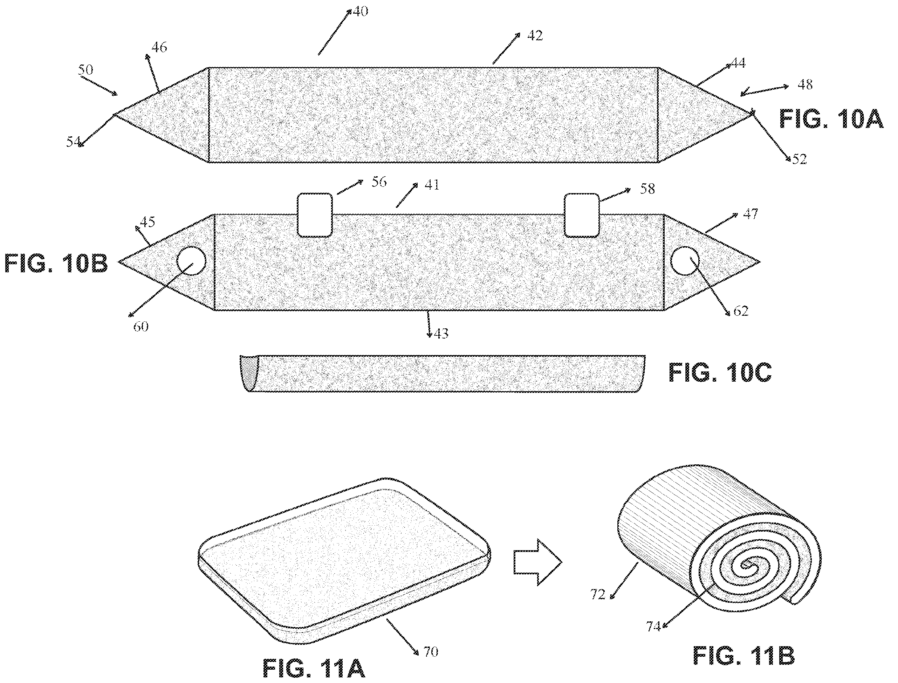

As illustrated in FIG. 1, a bone implant 10 is provided, the bone implant comprising, consisting essentially of, or consisting of a plurality of porous macroparticles 14 lyophilized into a rectangular shape 12 prepared from ceramic material and embedded in a matrix of collagen. The plurality of porous macroparticles are coated with a mineral coating. The mineral coating comprises, consists essentially of, or consists of a carbonated calcium-deficient hydroxyapatite component. The bone implant 10 also comprises a plurality of surface markers, for example, recesses, projections, or a combination thereof. In some embodiments, as illustrated in FIG. 1, bone implant 10 includes one or more channels 16 (which are recesses) which not only serve as surface markers but also increase the surface area and hydration characteristics of bone implant 10. Channels 16 are recesses that can have a macro half-cylindrical shape as illustrated in FIG. 10C. Channels 16 can have other shapes, for example, a trapezoidal or square shape, that can be easily identifiable under visible light. In some embodiments, hydration channel 16 can have a 1 mm diameter.

In various embodiments, the plurality of lyophilized porous macroparticles may include, but are not limited to, MasterGraft^{®} Strip produced by Medtronic Sofamor Danek, Inc., Memphis, Tenn.; MasterGraft^{®} Putty produced by Medtronic Sofamor Danek, Inc., Memphis, Tenn., and Matrix EXT compression resistant products produced by Medtronic Sofamor Danek, Inc., Memphis, Tenn.

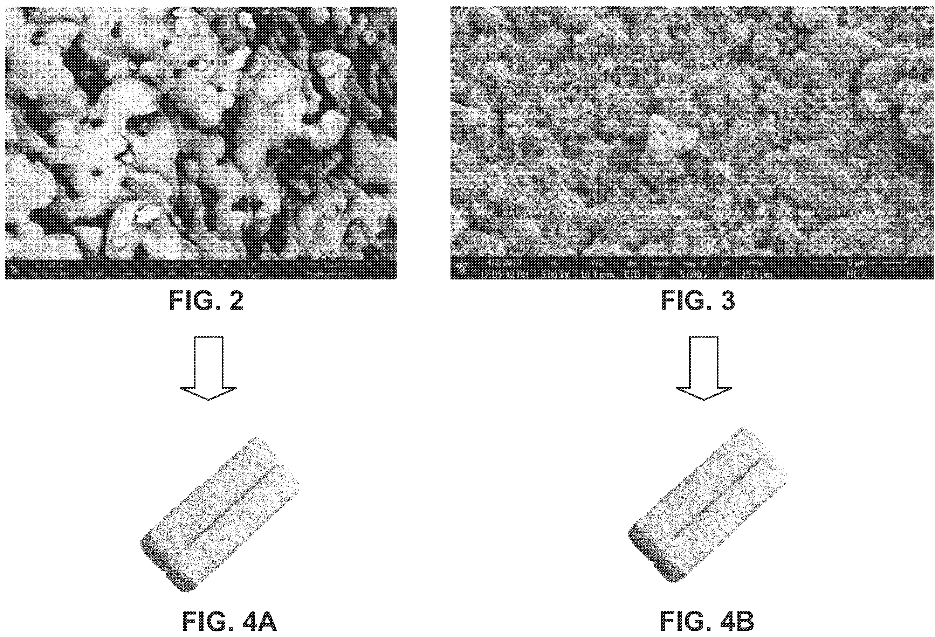

According to the claimed invention, the lyophilized porous macroparticles are coated with a mineral coating which comprises, consists essentially of, or consists of a plate-like nanostructure including a carbonate-substituted, calcium-deficient hydroxyapatite component. The plate-like nanostructure comprises nanoparticles having a size ranging from 10 to 100 nanometers. These mineral coatings are like bone and have been found to stimulate bone cells creating an enhanced cellular environment for bone healing. In these embodiments, the lyophilized porous macroparticles can be formed into strips or other resorbable osteoconductive cohesive scaffolds. While easily discernable under scanning electron microscope (SEM), the strips or scaffolds coated with the mineral coating are not readily distinguished under visible light. In sum, to the naked eye, whether the strips are or are not coated, visually, they may appear identical. For example, FIGS. 2 and 3 illustrate uncoated and coated lyophilized porous macroparticles, respectively. As illustrated in FIGS. 4A and 4B, strips prepared from either one of these materials visually appear identical. There is therefore a need to distinguish between these materials which is met as described in this application. In various embodiments, the goal of distinguishing between coated and uncoated macroparticles is accomplished by utilizing surface markers. It has also been found unexpectedly that the surface markers used to texturize the coated macroparticles serve not only to differentiate between coated and uncoated grafts but also increase the surface area of the coated macroparticles, exhibit increasing hydration characteristics and enhanced mechanical characteristics of a resulting implant allowing for improved biological integration into a selected surgical site. For example, while the surface area of a MasterGraft^{®} bone strip is from about 0.3 to about 0.5 m²/g, the surface area of a MasterGraft^{®} bone strip coated with a mineral coating comprising nanoparticles having carbonate substituted, calcium-deficient hydroxyapatite including surface markers provides an increase in surface area of from about 300 to about 500% to a surface area of from about 2, 3, 4, 5 m²/g, 6, 7, 8, 9, 10, 15, 20, 25, 30, 35, 40, 45, 50, 55, 60, 65, 70, 75, 80, 85, 90, 95 to about 100 m²/g.

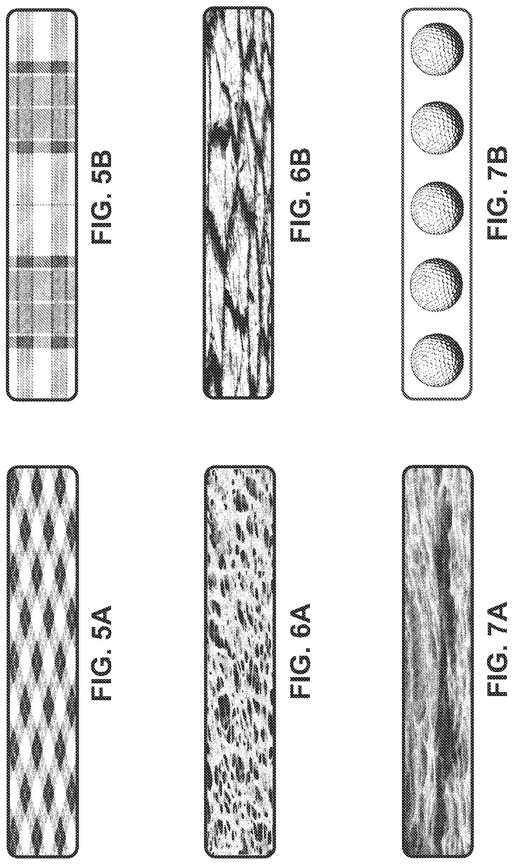

In various embodiments, the surface markers include, without limitation, macro texture features formed by applying texture patterns to one side of the bone implant formed in the mold surface or by cutting and etching texture patterns on most surfaces of the bone implant after lyophilization. For example, in some embodiments illustrated in FIGS. 5A, 5B, 6A, 6B, 7A and 7B, the macro texture features can include classic patterns such as rhomboidal (FIG. 5A) or plaid quilt (FIG. 5B), bone-like (FIGS. 6A, 6B and 7A), sphere, or golf ball patterns wherein each ball has a honeycomb indentation or oval design (FIG. 7B). These designs can be the macro structures in patterns of recesses and/or projections of a variety of shapes.

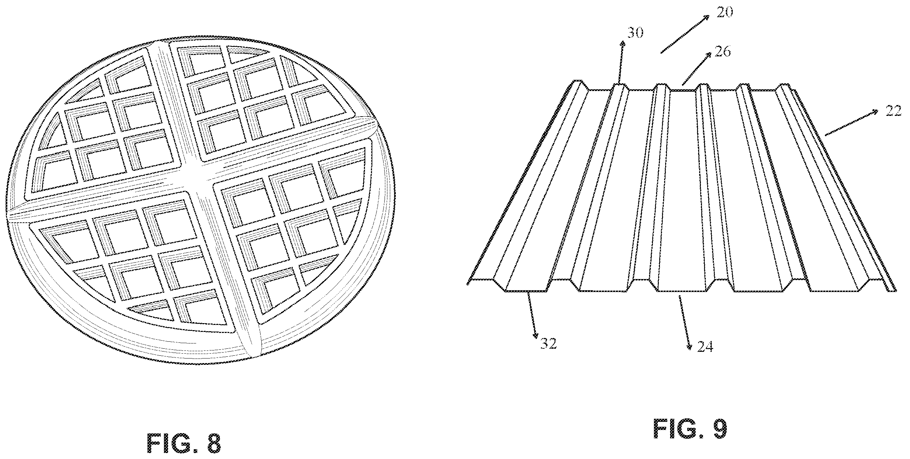

In some embodiments, macro surface markers include a plurality of recesses, projections, or a combination thereof, for example, squared or curved surfaces and various types of engineered notches. In various embodiments, the recesses, projections, or combinations thereof form a pattern or random shapes. In some aspects, the recesses, projections or combinations thereof form square, cylindrical, corrugated, notched, curved, waffle, hexagonal or honeycomb or oval shapes in the mineral coated bone implants. As illustrated in FIGS. 8, 9, 10A, 10B, 10C, 11A and 11B, the macro surface markers on the bone implants can include designs such as a waffle (FIG. 8), corrugated (FIG. 9), rectangular with triangular shapes (FIG. 10A), rectangular with triangular shapes and rectangular shaped notches (FIG. 10B) or curvatures as found in a cylindrical or semi-cylindrical shape (FIG. 10C). In other embodiments, unit cells and indentations such as hexagonal honeycomb design (FIG. 7B) can also be used as surface markers. In other embodiments, the plurality of recesses, projections or combinations thereof are radiographic markers, hydration channels, or indicia to determine bone growth within or bone growth adjacent to the bone implant.

FIG. 9 illustrates one embodiment of a mineral coated bone implant 20 having a corrugated shape. Bone implant 20 has a body 22 having a proximal end 24 and a distal end 26. Between proximal end 24 and distal end 26, body 22 of bone implant 20 contains a plurality of projections 30 and recesses 32 disposed in an alternating pattern to provide a corrugated design throughout body 22 of bone implant 20.

FIG. 10A illustrates an embodiment of a mineral coated bone implant 40 which has a rectangular body 42 having a triangular portion 44 at proximal end 48 and another triangular portion 46 at distal end 50. Each triangular shape 44 and 46 has an apex 52 and 54. FIG. 10B illustrates another embodiment of a mineral coated bone implant 41, wherein each triangular portion 45 and 47 contains a circular hole 60 and 62 which can be used to anchor bone implant 41 at a surgical site by using, for example, a screw (not shown). Body 43 of bone implant 41 can contain two rectangular notches 56 and 58, which can also be used to anchor or fit the bone implant at a surgical site.

FIG. 10C illustrates yet another mineral coated bone implant having a semi cylindrical shape. These various macro sized surface markers enable the surgeon to distinguish between mineral coated and uncoated bone implants as well as provide enhanced hydration and mechanical properties to the coated bone implants.

Other macro sized surface markers are illustrated in FIGS. 11A and 11B where a thin rectangle shaped strip 70 (FIG. 11A) can be rolled into a Swiss roll cake-like shape 72 (FIG. 11B) filled with, for example, autologous bone 74. This kind of surface marker allows for concentric radiopaque layers identifiable on radiograph to be incorporated into the bone implant. The macro sized surface markers provide for simplified radiographic identification and enable a surgeon to monitor the progression of the healing process. Moreover, the macro sized surface markers enhance the surface area of the bone implant, its hydration characteristics, and mechanical properties (e.g., flexibility) allowing for increased biological integration at the surgical site.

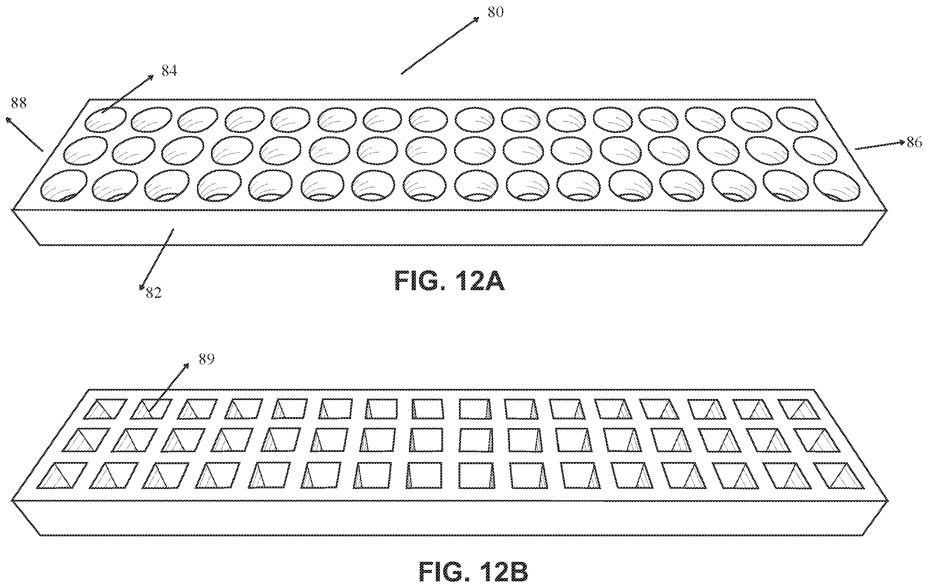

It will be understood that the notches, through circular holes, oval (not shown), square, rectangular, honeycomb designs can be disposed uniformly throughout the implant in a repeating pattern designs. For example, FIGS. 12A and 12B illustrate a repeating pattern of circle and square design macro surface markers, which enhance hydration, create space for bonding of the bone implant with the surrounding tissue and result in enhanced mechanical properties of the bone implant. In FIG. 12A, the mineral coated bone implant 80 has a body 82 with circle patterns 84 from proximal end 86 to distal end 88, wherein the circular pattern can be used not only as distinguishing surface markers but can also enhance the hydration of bone implant 80 and/or can be used to incorporate medicaments, for example. FIG. 12B illustrates a repeating pattern of square designs 89 which serve not only to distinguish the mineral coated bone implant but also to increase other properties of the bone implant, for example, hydration.

It will be understood that the surface markers can have an alternating pattern (e.g., circles alternating with squares, etc.) or can be disposed throughout the implant randomly in a nonuniform pattern.

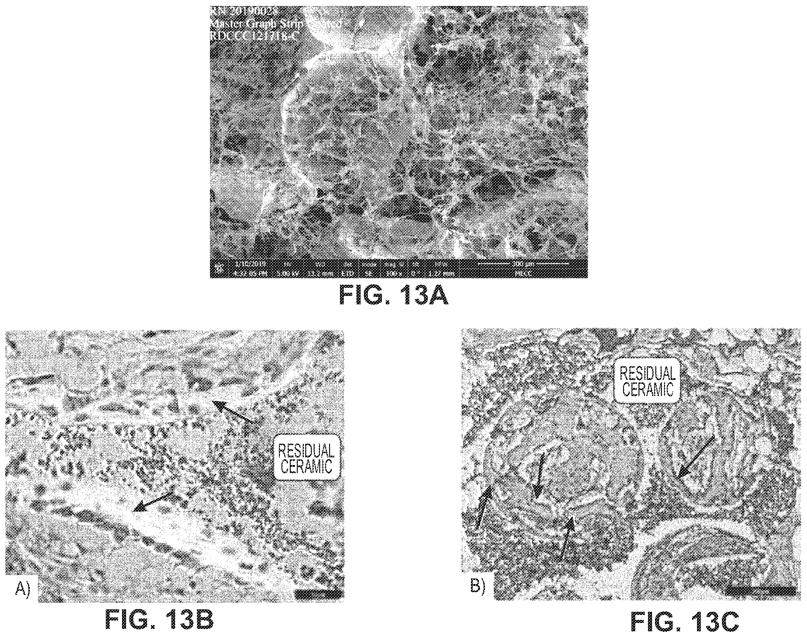

In various embodiments, other kinds of surface markers can be used, based on mesopores, micropores and/or nanopores, all visible by SEM radiography. For example, lyophilized porous macroparticles having mesopores allows cells to infiltrate throughout the bone implant thus providing additional space for new bone growth and vascularization. Mesopores include pores having a diameter from about 50 µm to about 1 mm. In some aspects, porous macroparticles having a porosity from about 200 to about 500 µm and up to about 5 mm have been found to provide increased vascularization. FIGS. 13A, 13B and 13C represent bone implants having mesopores. FIG. 13A illustrates a MasterGraph^{®} strip coated with the mineral coating as described in this application. After one week of implantation, magnified histological images at the periphery of the implant as illustrated in FIG. 13B show *de novo* bone deposition (arrows pointed to the left) at 200 times magnification. FIG. 13C illustrates osteoblasts (black arrows) lining the bone tissue directing growth away form the ceramic surface into the macropore space at 79 times magnification.

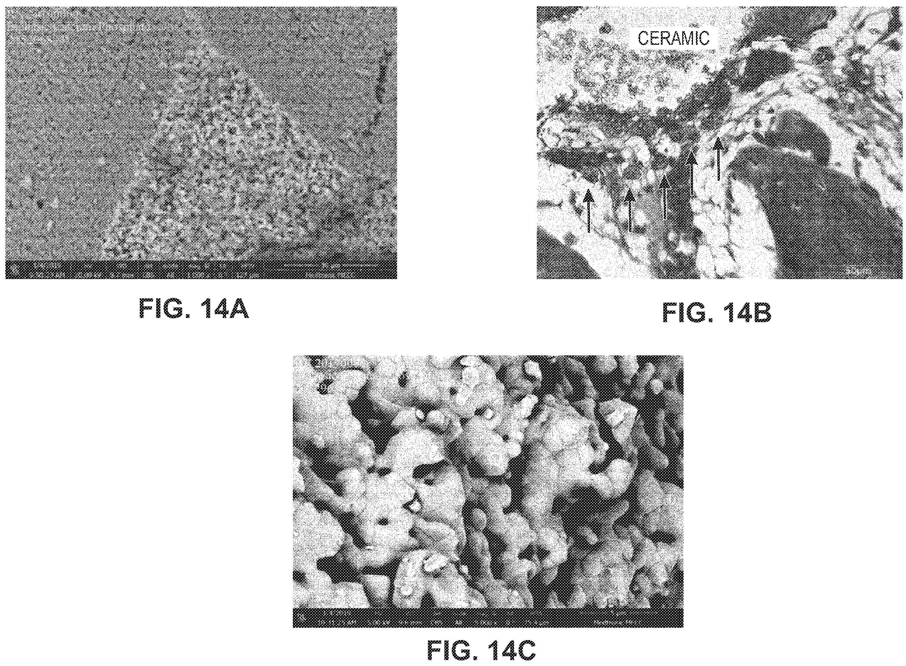

In various embodiments, bone implants prepared of uncoated MasterGraft^{®}, for example, can contain micropores of about 500 µm. The addition of a mineral coating having micropores increases the surface area of MasterGraft^{®} bone implant and enhances the dissolution rate of Ca²⁺ and PO₄⁻. As a result of the increase in the dissolution rate of Ca²⁺ and PO₄⁻ new mineral deposition of bone material increases. Further, micron-sized grains may be removed following cell-mediated resorption. FIGS. 14A, 14B and 14C illustrate uncoated (FIG. 14C), mineral coated (FIG. 14A), and cell-mediated resorption (black arrows in FIG. 14B) in porous macroparticles having micropores. In various embodiments, other kinds of bone implant can be mineral coated with coatings having surface markers as described in this application.

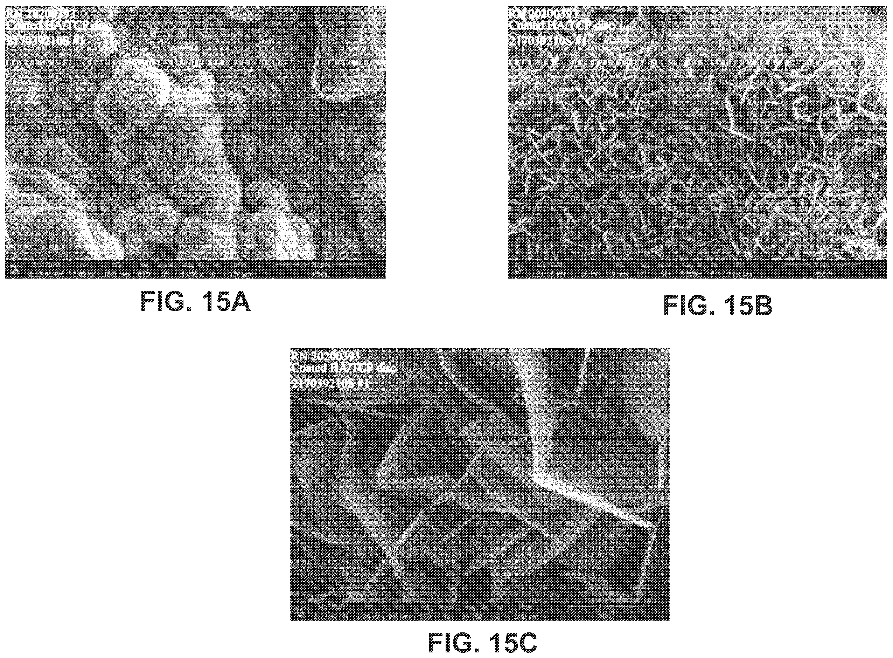

In embodiments according to the claimed invention, MasterGraft^{®} bone implants and other kinds of bone implants are coated with mineralized coatings having a plate-like nanostructure of carbonate substituted, calcium-deficient hydroxyapatite (HA/TCP) shown in different magnifications in FIG. 15A (1000x), FIG. 15B (5000x) and FIG. 15C (25,000x). The resulting nanopore size varies from about 0.1 µm to about 0.2 µm. In these embodiments, the nano morphology of the mineral coating increases the specific surface are of the bone implant and as a result it stimulates bone cell growth. In addition, the increased surface area further enhances early-stage dissolution increasing the availability of Ca²⁺ and PO₄⁻, thus increasing new mineral deposition.

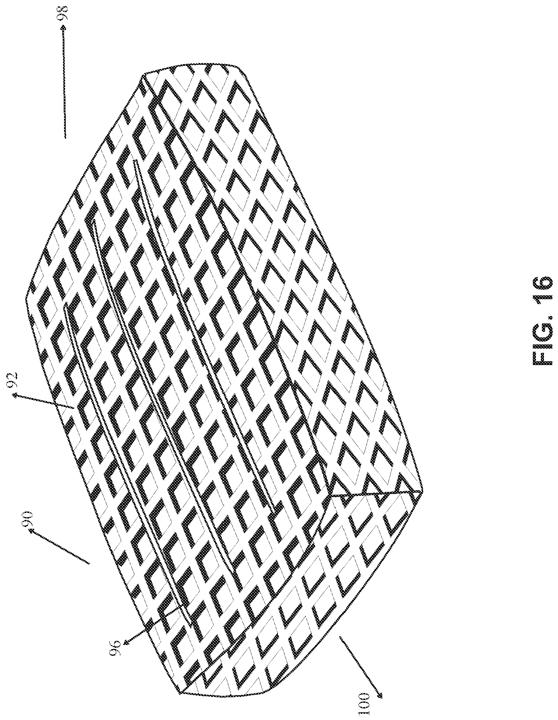

FIG. 16 illustrates another embodiment of a mineral coated bone implant having macro surface markers having a waffle design. FIG. 16 is a perspective view of one embodiment of the coated bone implant 90 of FIG. 1 including a plurality of lyophilized porous macroparticles coated with a mineral coating having macro surface markers that are recesses shaped as squares and projections bordering the squares, which create a waffle design 92, the design disposed uniformly throughout the coated implant between the proximal end 98 and the distal end 100 of the implant. Bone implant 90 includes one or more channels 96 which together with the waffle design increase the surface area and hydration characteristics of bone implant 90.

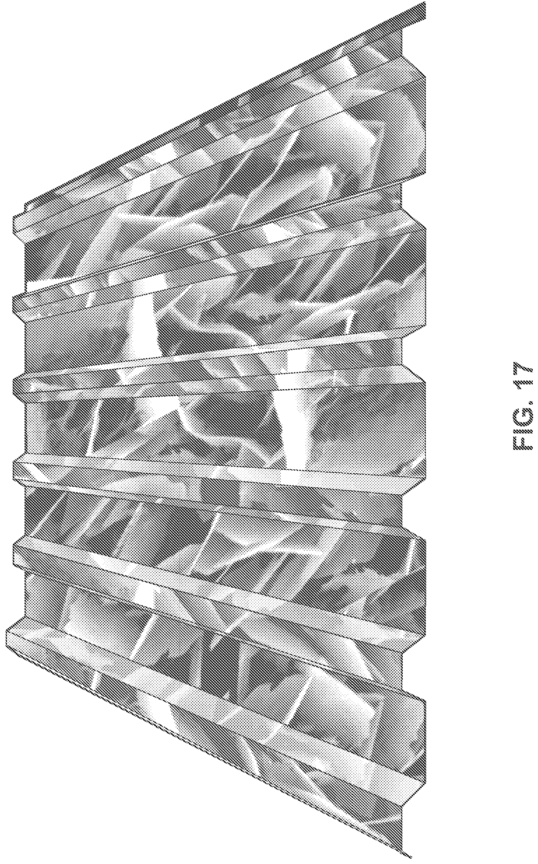

In another embodiment illustrated in FIG. 17, the nano morphology of mineralized coating can be applied to the macro surface marker of a corrugated shape bone implant. In several aspects, the bone implant has pores (i) from about 50 µm to about 500 µm for bone growth or (ii) from about 500 µm to about 5 mm for vascularization.

### Lyophilized Porous Macroparticles

The ceramic material can comprise synthetic ceramic or ceramics including hydroxyapatite and beta-tricalcium phosphate. The ceramic material can be in a powder form. The ceramic material comprises a calcium to phosphate ratio of between 1.0 to about 2.0. In some embodiments, the calcium to phosphate ratio is between 1.0, 1.1, 1.2, 1.3, 1.4, 1.5, 1.6, 1.7, 1.8, 1.9 to about 2.0.

The ceramic material of the lyophilized porous macroparticles is a biphasic calcium phosphate comprising hydroxyapatite in an amount of about 8 to about 22 wt. % and beta-tricalcium phosphate in an amount of about 78 to about 92 wt. %. In some embodiments, the hydroxyapatite is in an amount of about 8, 9, 10, 11, 12, 13, 14, 15, 16, 17, 18, 19, 20, 21 to about 22 wt. % and the beta-tricalcium phosphate in an amount of about 78, 79, 80, 81, 82, 83, 84, 85, 86, 87, 88, 89, 90, 91 to about 92 wt. %.

The porous ceramic particles comprise hydroxyapatite and beta-tricalcium phosphate. The hydroxyapatite is in an amount of about 8 to about 22 wt. % based on a total weight of a ceramic granule. The hydroxyapatite can be in a range from about 8, 9, 10, 11, 12, 13, 14, 15, 16, 17, 18, 19, 20, 21 to about 22 wt. %. In some embodiments, the hydroxyapatite can be in a range from about 1 to about 99 wt. %, such as from 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 to about 99 wt. %.

The beta-tricalcium phosphate is in an amount of about 78 to about 92 wt. % based on a total weight of a ceramic granule. The beta-tricalcium phosphate can be in an amount from about 78, 79, 80, 81, 82, 83, 84, 85, 86, 87, 88, 89, 90, 91 to about 92 wt. %. In some embodiments, the beta-tricalcium phosphate can be in a range from about 1 to about 99 wt. %, such as from 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 to about 99 wt. %.

The porous ceramic particles can have a calcium to phosphate ratio of between 1.0 to about 2.0. In some embodiments, the calcium to phosphate ratio is between 1.0, 1.1, 1.2, 1.3, 1.4, 1.5, 1.6, 1.7, 1.8, 1.9 to about 2.0.

In some embodiments, the lyophilized porous macroparticles comprise collagen-containing biomaterials from the implant market which, when placed in a bone defect, provide scaffolding around which the patient's new bone and/or cartilage will grow, gradually replacing the carrier matrix as the target site heals. In other embodiments, ceramic particles coated with mineral coatings can be embedded in a porous carrier matrix of cross-linked or noncross-linked collagen, that can also include identification or surface markers that distinguish them from uncoated ceramic particles. Examples of suitable lyophilized porous macroparticles may include, but are not limited to, the MasterGraft^{®} Matrix produced by Medtronic Sofamor Danek, Inc., Memphis, Tenn.; MasterGraft^{®} Putty produced by Medtronic Sofamor Danek, Inc., Memphis, Tenn. The MasterGraft^{®} Matrix or the MasterGraft^{®} Putty are synthetic bone grafts containing biphasic calcium phosphate (85% beta-tricalcium phosphate (β-TCP) and 15% hydroxyapatite (HA) in porous granules alone or in combination with bovine type I collagen. In some embodiments, each macroparticle contains 15 % hydroxyapatite and 85% beta-tricalcium phosphate (β-TCP). This ratio provides long term stability and a good resorption rate. In other embodiments, each macroparticle can contain 2.5% +/- 0.25% collagen and 97.5% +/- 0.25% β-TCP.

The polymer component of each of the plurality of lyophilized porous macroparticles can be porcine or bovine collagen, bovine type I collagen, tendon or dermis derived collagen, or a combination thereof.

Each of the plurality of lyophilized porous macroparticles have an average diameter from about 0.1 mm to about 10 mm. For example, each of the lyophilized porous macroparticles can have an average diameter from about 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9 to about 10 mm. In some embodiments, the lyophilized porous macroparticle can have a granule size from about 0.09 mm to about 0.6 mm.

Each of the lyophilized porous macroparticles can have an average height and/or length from about 0.05, 0.01 mm to about 10 mm or from about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9 to about 10 mm.

The ceramic material provided in each of the lyophilized porous macroparticles can be in the form of porous ceramic granules. The porous ceramic granules are like the granules found and described in U.S. Application Serial No. 16/523,259, filed on July 26, 2019, assigned to Warsaw Orthopedic, Inc.. The porous ceramic granules have an average diameter from about 50 µm to 1.6 mm. In some embodiments, the average diameter of the granules may be from about 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, 500, 505, 510, 515, 520, 525, 530, 535, 540, 545, 550, 555, 560, 565, 570, 575, 580, 585, 590, 595, 600, 605, 610, 615, 620, 625, 630, 635, 640, 645, 650, 655, 660, 665, 670, 675, 680, 685, 690, 695, 700, 705, 710, 715, 720, 725, 730, 735, 740, 745, 750, 755, 760, 765, 770, 775, 780, 785, 790, 795, 800, 850, 900, 950, 1000, 1100, 1150, 1200, 1250, 1300, 1350, 1400, 1450, 1500, 1550 to about 1600 µm. It is to be understood that the ceramic material is smaller in size than the macroparticles.

When the porous ceramic granules are used in the composition, the granules are in an amount from about 50 to about 98 wt. % and the collagen is in an amount from about 2 to about 50 wt. % based on a total weight of each of the lyophilized porous macroparticles. Each of the porous ceramic granules comprise hydroxyapatite and beta-tricalcium phosphate having a calcium to phosphate ratio of between 1.0 to about 2.0, as described above regarding the ceramic material.

Each of the porous ceramic granules have a Brunauer-Emmett Teller (BET) surface area from about 0.2 to about 10 m²/g. The BET surface area can be from about 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 to about 100 m²/g. The increase in surface area further facilitates new bone growth by allowing the granules to dissolve and release calcium faster than a granule would.

Each of the porous ceramic granules can have a microporosity, and the diameter of the micropores is from about 0.01 to about 10 microns. In some embodiments, the diameter of each of the micropores can be from about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9 to about 10 microns. In some embodiments, the median pore diameter can be about 125 µm and the average pore diameter can be 78 µm.

The plurality of lyophilized porous macroparticles can be made into a variety of shapes after lyophilization or using cryogel applications. The shapes can be cut from a textured or flat shaped sheet of bone material comprising the ceramic material and collagen. The macroparticles are porous, but in some embodiments, the macroparticles are highly porous. For example, porous macroparticles can include that the macroparticles have a porosity from about 10 to about 80% or from about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 to about 80%. Highly porous macroparticles can include that the macroparticles have a porosity from about 81 to about 99% or from about 81, 82, 83, 84, 85, 86, 87, 88, 89, 90, 91, 92, 93, 94, 95, 96, 97, 98 to about 99%.

The macroparticle shapes and size create a high level of surface area which increases uniform hydration when fluid is administered to the macroparticles. For example, due to the high level of surface area, fluid will rapidly move into the macroparticles through wicking. The macroparticle shapes can include cylinders; cubes; rods or tubes; a hollow tube or tubes; a half hemispherical hollow tube or tubes; a rectangle or rectangles; a disc or discs; electro-spun fibers or a combination thereof.

The plurality of lyophilized porous macroparticles in the chamber can have a packing density to maximize hydration of the plurality of lyophilized porous macroparticles when fluid is introduced. In some embodiments, the packing density can be high. The packing density of a MasterGraft ^{®} product can be from about 1 to about 5 g/cm³, from about 1 to about 4 g/cm³, from about 1 to about 3 g/cm³, or from about 1 to about 2 g/cm³. The packing density can be from about 1, 2, 3, 4 to about 5 g/cm³. In some embodiments, the packing density of the macroparticles combined with the high surface area of the macroparticles, creates a uniformly hydrated composition that does not agglomerate and is flowable.

The fluid used to hydrate the macroparticles can include bone marrow aspirate, saline, sterile water, blood for injection, phosphate buffered saline, dextrose, Ringer's lactated solution, or a combination thereof. The ratio of fluid to the plurality of lyophilized porous macroparticles can be from about 0.5:1 to about 3:1. In some embodiments, the ratio of fluid to the plurality of lyophilized porous macroparticles can be from about 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 2.6:1, 2.7:1, 2.8:1, 2.9:1 to about 3:1.

Autograft bone can be added to the hydratable composition before or after hydration. The autograft bone can be cut into various shapes, including fibers, chips, granules, powder, shards, shavings, or a combination thereof. The autograft bone can be cut into specific sizes. For example, the autograft bone can be from about 1 to about 10 mm. In some embodiments, the size of the autograft bone added to the composition can be from about 1, 2, 3, 4, 5, 6, 7, 8, 9 to about 10 mm. In some embodiments, the autograft bone is cut into bone chips having a size from about 1 to about 4 mm and is added to the hydratable composition after hydration.

A certain amount of autograft bone can be added to the hydratable composition, such as from about 0 to about 50 vol. % or from about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 to about 50 vol. % based on the total weight of the hydratable composition. In some embodiments, the composition can contain greater than 50 vol. % of autograft bone without the composition losing its cohesive properties.

In some embodiments, the hydratable composition has a flowable viscosity starting from about 50 Pascal-second (Pa-s), 100 Pa-s, 150 Pa-s, 200 Pa-s, 250 Pa-s, to about 300 Pa-s and reaches a higher viscosity from about 500 Pa-s, 750 Pa-s, 1000 Pa-s, 1,500 Pa-s, 2,000 Pa-s, 2,500 Pa-s to about 3,000 Pa-s. In some embodiments, the hydratable composition has a flowable viscosity starting from about 50 Pa-s to about 3,000 Pa-s and reaches a higher viscosity from about 3,000 Pa-s to about 300,000 Pa-s.

The hydratable composition can have a certain density when hydrated. For example, when the composition is hydrated, the density can be from about 1.2 to about 2.0 g/cc or from about 1.4 to about 1.6 g/cc. In some embodiments, the hydrated composition can have a density from about 1.2, 1.3, 1.4, 1.5, 1.6, 1.7, 1.8, 1.9 to about 2.0 g/cc.

The hydratable composition can have a modulus of elasticity from about 2 MPa to about 12 MPa, such as from about 2, 3, 4, 5, 6, 7, 8, 9, 10, 11 to about 12 MPa.

In some embodiments, if autograft bone is added to the composition after hydration, the modulus of elasticity can be increased with the addition of the autograft bone.

In some embodiments, the fluid used to hydrate the composition can include sterile water, saline, phosphate buffered saline (PBS), hyaluronic acid, cellulose ethers (such as carboxymethyl cellulose), water, collagen, gelatin, autoclaved bone powder, osteoconductive carriers, whole blood, blood fractions, concentrated bone marrow aspirate, and mixtures thereof. Non-limiting examples of blood fractions include serum, plasma, platelet-rich plasma, concentrated platelet-rich plasma, platelet-poor plasma, and concentrated platelet poor plasma.

In some embodiments, additional materials may be added to the composition such as one or more of poly (alpha-hydroxy acids), polyglycolide (PG), polyethylene glycol (PEG) conjugates of poly (alpha-hydroxy acids), polyorthoesters (POE), polyaspirins, polyphosphagenes, gelatin, hydrolyzed gelatin, fractions of hydrolyzed gelatin, elastin, starch, pre-gelatinized starch, hyaluronic acid, chitosan, alginate, albumin, fibrin, vitamin E analogs, such as alpha tocopheryl acetate, d-alpha tocopheryl succinate, D,L-lactide, or L-lactide, caprolactone, dextrans, vinylpyrrolidone, polyvinyl alcohol (PVA), PVA-g-PLGA, PEGT-PBT copolymer (polyactive), methacrylates, PEO-PPO-PAA copolymers, PLGA-PEO-PLGA, PEG-PLG, PLA-PLGA, poloxamer 407, PEG-PLGA-PEG triblock copolymers, POE, SAIB (sucrose acetate isobutyrate), polydioxanone, methylmethacrylate (MMA), MMA and N-vinylpyyrolidone, polyamide, oxycellulose, copolymer of glycolic acid and trimethylene carbonate, polyesteramides, polyether ether ketone, polymethylmethacrylate, silicone, hyaluronic acid, or combinations thereof.

In some embodiments, the macroparticles can in addition to collagen comprise at least one biodegradable polymer comprising one or more of poly(lactide-co-glycolide) (PLGA), polylactide (PLA), polyglycolide (PGA), D-lactide, D,L-lactide, L-lactide, D,L-lactide-co-ε-caprolactone, L-lactide-co-ε-caprolactone, D,L-lactide-co-glycolide-co-e-caprolactone, poly(D,L-lactide-co-caprolactone), poly(L-lactide-co-caprolactone), poly(D-lactide-co-caprolactone), poly(D,L-lactide), poly(D-lactide), poly(L-lactide), poly(esteramide), carboxymethylcellulose (CMC), alkylene oxide copolymer (AOC) or a combination thereof.

The macroparticles comprise in addition to collagen at least one ceramic material, including, but not limited to synthetic ceramics selected from one or more materials comprising calcium phosphate ceramics or silicon ceramics. Biological glasses such as calcium-silicate-based bioglass, silicon calcium phosphate, tricalcium phosphate (TCP), biphasic calcium phosphate, calcium sulfate, hydroxyapatite, coralline hydroxyapatite, silicon carbide, silicon nitride (Si₃N₄), and biocompatible ceramics may be used. In some embodiments, the ceramic material is tri-calcium phosphate or biphasic calcium phosphate and silicon ceramics. In some embodiments, the ceramic material is tricalcium phosphate. The macroparticles can further include materials such as demineralized bone matrices (DBMs), stem cells, growth factors or combinations thereof.

In some embodiments, the ceramic material is a combination of a calcium phosphate ceramic and a silicon ceramic. In some embodiments, the calcium phosphate ceramic is resorbable biphasic calcium phosphate (BCP) or resorbable tri-calcium phosphate (TCP).

The ceramic material of the disclosure may also be oxide ceramics such as alumina (Al₂O₃) or zirconia (ZrO₂) or composite combinations of oxides and non-oxides such as silicon nitride.

In various embodiments, the plurality of lyophilized porous macroparticles comprise ceramic material in an amount from about 50 to about 98 wt. % and collagen in an amount from about 2 to about 50 wt. % based on a total weight of each of the lyophilized porous macroparticles, each of the lyophilized porous macroparticles having an average diameter from about 0.1 mm to about 10 mm. In other embodiments the bone implant described in this application is rollable and has a thickness of from about 1 mm to about 4 mm and comprises up to 83% ceramic material. In some embodiments, the bone implant has a surface area of at least about 5 m²/g, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 to about 100 m²/g.

### Coatings

The mineral coating of the lyophilized porous macroparticles, as described herein, can be carried out by any conventional manner. A useful mineral coating can be prepared as described in U.S. Application Ser. No. 15/060,547 filed March 3, 2016 and published as US20160271296A1 and U.S. Application Ser. No. 13/879,178 filed September 25, 2009 and published as US20140161886A1. These applications provide mineral coating having a plate-like nanostructure to the implant having the macro surface markers (e.g., recesses and/or projections). According to the claimed invention, the plate-like nanostructure comprises nanoparticles having a size range from 10 to 100 nanometers.

As described in these patent applications, the mineral coating can be calcium-containing. According to the claimed invention, the mineral coating comprises a nano-sized feature comprising a carbonate-substituted, calcium-deficient hydroxyapatite component. For example, the calcium-containing mineral coating can include hydroxyapatite (HAP), α-tricalcium phosphate (α-TCP), β-tricalcium phosphate (β-TCP), amorphous calcium phosphate, dicalcium phosphate, octacalcium phosphate, calcium phosphate (CaP), or calcium carbonate. The calcium-containing mineral coating can comprise a plurality of layers, e.g., separate layers having distinct dissolution profiles. Under physiological conditions, solubility of calcium phosphate species can adhere to the following trend: amorphous calcium phosphate>dicalcium phosphate>octacalcium phosphate>β-TCP>HAP. A dicalcium phosphate mineral can have a dissolution rate that is more than fifty times higher than that of HAP. Therefore, creation of a matrix with distinct calcium phosphate layers allows for a broad range of dissolution patterns.

The mineral coating of the lyophilized porous macroparticles, can be provided by incubating the macroparticles. For example, in some embodiments, the mineral coating, described herein, can be developed by incubating the constituents in modified simulated body fluid (mSBF), for four days or more at a pH of about 6.8 to about 7.4 and at a temperature of about 37° C. The SBF or mSBF can be refreshed daily. This procedure produces a calcium-deficient, carbonate-containing apatite material on alginate and on poly-(α-hydroxy esters) as described in U.S. Pat. No. 6,767,928. mSBF can include elevated calcium and phosphate. In general, an increase in pH can favor hydroxyapatite growth, while a decrease in pH can favor octacalcium phosphate mineral growth.

In other embodiements, conditions favorable for hydroxyapatite formation can include a pH between about 5.0 and about 8.0 and a calcium concentration multiplied by a phosphate concentration between about 10⁻⁵ and about 10⁻⁸ M. Conditions favorable for octacalcium phosphate formation include a pH between about 6.0 and about 8.0 and a calcium concentration multiplied by a phosphate concentration between about 10⁻⁵ and about 10^{-7.5} M. Furthermore, conditions favorable for dicalcium phosphate dehydrate formation can include a pH between about 6.0 and about 8.0 and a calcium concentration multiplied by a phosphate concentration between about 10⁻⁴ and about 10⁻⁶ M.

In yet other embodiments, the pH of mSBF can be varied between about 5.0 and about 6.0 to promote hydroxyapatite formation. Similarly, the pH of mSBF can be varied between about 6.0 and about 6.5 to promote octacalcium phosphate and hydroxyapatite formation. Likewise, the pH of mSBF can be varied between about 6.5 and about 8.0 to promote dicalcium phosphate, octacalcium phosphate, and hydroxyapatite formation.

The mineral coating useful for the lyophilized porous macroparticles of the bone implant described in this application can be similar in structure and composition to human bone mineral. For example, the mineral coating can include spherical clusters with the plate-like structure and the carbonate-substituted, calcium-deficient hydroxyapatite phase composition.

As another example, the mineral coating can include an apatite. Apatite can include calcium phosphate, calcium carbonate, calcium fluoride, calcium hydroxide, or citrate.

As another example, a mineral coating can comprise a plurality of discrete mineral islands on the scaffold, or the mineral coating can be formed on the entire surface of the scaffold. As another example, the mineral coating can comprise a substantially homogeneous mineral coating. In other embodiments, the mineral coatings can comprise any suitable coating material containing calcium and phosphate, such as hydroxyapatite, calcium-deficient carbonate-containing hydroxyapatite, tricalcium phosphate, amorphous calcium phosphate, octacalcium phosphate, dicalcium phosphate, calcium phosphate, anorganic bone or a mixture thereof. According to the claimed invention, the osteoconductive mineral coating at least comprises a calcium-deficient carbonate-containing hydroxyapatite.

According to the claimed invention, the mineral coating includes carbonate-substituted, calcium-deficient hydroxyapatite. hydroxyapatite. Calcium-deficient (non-stochiometric) hydroxyapatite, Ca_{10-*x*}(PO₄)_{6-*x*}(HPO₄)*ₓ*(OH)_{2-*x*} (where x is between 0 and 1) has a Ca/P ratio between 1.67 and 1.5. The Ca/P ratio is often used in the discussion of calcium phosphate phases. Stoichiometric apatite Ca₁₀(PO₄)₆(OH)₂ has a Ca/P ratio of 10:6 normally expressed as 1.67. The non-stoichiometric phases have the hydroxyapatite structure with cation vacancies (Ca²⁺) and anion (OH⁻) vacancies. Hydroxyapatite can be predominantly crystalline, but, in some cases, may be present in amorphous forms. The mineral coatings useful in this application can include from at least about 1% to at least about 100% hydroxyapatite, including the discrete values included in this range.

It is well known that the hydroxyapatite within the bones of living organisms are very thin plate-like carbonate structures which have an average of 50 nm length, 2-3 nm thickness and a width of 25 nm. (Heliyon, Volume 6, Issue 4, 2020, Article e03655)

In some embodiments, the mineral coating can include octacalcium phosphate. Octacalcium phosphate has a chemical formula of Ca₈H₂(PO₄)_{6 •}5H₂O or can also be written as Ca₄HO₁₂P₃. Octacalcium phosphate has been shown to be a precursor of hydroxyapatite. Hydrolysis of octacalcium phosphate can create hydroxyapatite. Octacalcium phosphate can be predominantly crystalline, but, in some cases, may be present in amorphous forms. The mineral coating, in some aspects, can include at least about 1% to at least about 100% octacalcium phosphate including the discrete values included in this range.

The mineral coating useful for coating the lyophilized porous macroparticles can include at least about 1% or at least about 100% porosity including all discrete values included in this range. The mineral coating can also include a pore diameter from about 1 nm to about 3500 nm including all discrete values included in this range. The mineral coating useful in this application can include a ratio of at least about 0.1 Ca/P to about 10 Ca/P including the discrete values included in this range.

The mineral coating covering the plurality of lyophilized porous macroparticles comprises a plate-like nanostructure comprising nanoparticles having a size range from 10 to 100 nanometers. The mineral coating comprises a carbonate-substituted, calcium-deficient hydroxyapatite component. In some embodiments, the mineral coating further comprises a calcium-containing mineral, the calcium-containing mineral is at least one of apatite, hydroxyapatite, α-tricalcium phosphate, β-tricalcium phosphate, amorphous calcium phosphate, dicalcium phosphate, octacalcium phosphate, calcium carbonate, anorganic bone or combinations thereof. As used herein anorganic bone refers to bone mineral only, with the organic constituents removed. In other embodiments, the plurality of lyophilized porous macroparticles comprise tricalcium phosphate and hydroxyapatite.

### Methods of Making the Bone Implant

A method of making the implant described in this application is provided. The method includes providing a plurality of porous macroparticles comprising ceramic material; coating the plurality of porous macroparticles with a mineral coating; adding the plurality of coated porous macroparticles to a collagen slurry to form a mixture. The mineral coating of the porous macroparticles comprises nanoparticles having a carbonate-substituted, calcium-deficient hydroxyapatite component, wherein the mineral coating comprises a plate-like nanostructure comprising nanoparticles having a size range from 10 to 100 nanometers; and lyophilizing the mixture comprising the macroparticles to form the bone implant comprising a plurality of recesses, projections or a combination thereof. In various embodiments, the lyophilizing of the mixture comprising the nanoparticles occurs in a mold to form square, cylindrical, corrugated, notches, curves, waffles, or hexagonal honeycomb shapes of the recesses and/or projections of the bone implant. In some embodiments, the plurality of recesses, projections or a combination thereof are etched into the implant. In many aspects, the plurality of recesses, projections or a combination thereof are radiographic markers, hydration channels, or indicia to determine bone growth within or bone growth adjacent to the bone implant. In some embodiments, the plurality of porous particles comprises ceramic material in an amount from about 50 to about 98 wt. % and collagen in an amount from about 2 to about 50 wt. % based on a total weight of each of the lyophilized porous macroparticles, each of the lyophilized porous macroparticles having an average diameter from about 0.1 mm to about 10 mm.

The collagen used in the slurry can be from skin, tendon, fascia, ligament, trachea, or organ collagen. In certain embodiments, the collagen is human collagen or another mammalian collagen (e.g., porcine, bovine, or ovine). The collagen can be sourced from any animal.

Generally, there are about twenty-eight distinct collagen types that have been identified in vertebrates, including bovine, ovine, porcine, chicken, marine, and human sources. The collagen types are numbered by Roman numerals, and the chains found in each collagen type are identified by Arabic numerals. Detailed descriptions of structure and biological functions of the various types of naturally occurring collagens are generally available in the art.

The collagen may have the same composition as in naturally occurring sources. Examples of sources of collagens include human or non-human (bovine, ovine, and/or porcine), as well as recombinant collagen or combinations thereof. Examples of suitable collagen include, but are not limited to, human collagen type I, human collagen type II, human collagen type III, human collagen type IV, human collagen type V, human collagen type VI, human collagen type VII, human collagen type VIII, human collagen type IX, human collagen type X, human collagen type XI, human collagen type XII, human collagen type XIII, human collagen type XIV, human collagen type XV, human collagen type XVI, human collagen type XVII, human collagen type XVIII, human collagen type XIX, human collagen type XXI, human collagen type XXII, human collagen type XXIII, human collagen type XXIV, human collagen type XXV, human collagen type XXVI, human collagen type XXVII, and human collagen type XXVIII, or combinations thereof. Collagen may further or alternatively comprise hetero- and homo-trimers of any of the above-recited collagen types. In some embodiments, the collagen comprises hetero- or homo-trimers of human collagen type I, human collagen type II, human collagen type III, or combinations thereof. In some embodiments, the collagen is type I or substantially all is collagen type I, e.g., at least 60%, at least 65%, at least 70%, at least 75%, at least 80%, at least 85%, at least 90%, or at least 95%.

The method of providing a collagen slurry or suspension includes mixing the slurry or suspension with the plurality of porous macroparticles coated with a mineral coating under conditions sufficient to cause the collagen slurry to self-assemble into macroscopic collagen fibers and cause the plurality of coated porous macroparticles to form a collagen mixture and lyophilizing the mixture into macroparticles to form the bone implant. Conditions sufficient to cause the collagen slurry to self-assembly comprise raising the pH of the slurry to from about 5 to about a pH of 9 and/or adding bone powder, calcium phosphate, hydroxyapatite, or a mixture thereof to the slurry to raise the pH from about 5 to about 9, placed into a mold and lyophilized or freeze dried into final coated forms. In various embodiments, the mold includes surface markers which provide macro, meso, micro or nano texture markers as described above.

Also described herein is a method for producing mineral coated porous macroparticles including (i) contacting the porous macroparticles with a modified simulated body fluid; and (ii) incubating the porous macroparticles and the modified simulated body fluid for a period of time under conditions sufficient to form mineral coated porous macroparticles, wherein the porous macroparticles comprise ceramic material and collagen; and the mineral coating comprises a plate-like nanostructure and a carbonate-substituted, calcium-deficient hydroxyapatite component.

A method of treating a bone defect, which is not part of the claimed invention, is described in the following. The method of treatment comprises implanting a bone implant into the defect, the bone implant comprising a plurality of lyophilized porous macroparticles comprising ceramic material and collagen, the plurality of lyophilized porous macroparticles coated with a mineral coating, the mineral coating comprising nanoparticles comprising a carbonate-substituted, calcium-deficient hydroxyapatite component, the bone implant comprising a plurality of recesses, projections or a combination thereof. The bone implant is hydrated with fluid comprising bone marrow aspirate, saline, sterile water, blood for injection, phosphate buffered saline, dextrose, Ringer's lactated solution, or a combination thereof before, during or after the bone implant is implanted into the bone defect.

## Claims

1. A bone implant (10) comprising a plurality of lyophilized porous macroparticles (14) comprising ceramic material and collagen, the plurality of lyophilized porous macroparticles (14) coated with a mineral coating, the mineral coating comprising a nano-sized feature comprising a carbonate-substituted, calcium-deficient hydroxyapatite component, the bone implant (10) comprising a plurality of recesses, projections or a combination thereof,
wherein the mineral coating comprises a plate-like nanostructure comprising nanoparticles having a size range from 10 to 100 nanometers.

2. The bone implant (10) of claim 1, wherein the recesses, projections or a combination thereof form square, cylindrical, corrugated, notches, curve, waffle, hexagonal honeycomb shapes in a pattern or randomly about the bone implant (10).

3. The bone implant (10) of claim 2, wherein the plurality of recesses, projections or a combination thereof are radiographic markers, hydration channels, or indicia to determine bone growth within or bone growth adjacent to the bone implant (10).

4. The bone implant (10) of claim 1, wherein the plurality of lyophilized porous macroparticles (14) comprise ceramic material in an amount from about 50 to about 98 wt. % and collagen in an amount from about 2 to about 50 wt. % based on a total weight of each of the lyophilized porous macroparticles (14), each of the lyophilized porous macroparticles (14) having an average diameter from about 0.05 mm to about 10 mm.

5. The bone implant (10) of claim 1, wherein the bone implant (10) is rollable and has (i) a thickness of from about 1 mm to about 4 mm and comprises up to 83% ceramic material; and/or (ii) a surface area of at least about 1 m²/g to about 100 m²/g.

6. The bone implant (10) of claim 1, wherein the bone implant (10) has pores (i) from about 50 microns to about 500 microns for bone growth; or (ii) from about 500 microns to about 5 mm for vascularization.

7. The bone implant (10) of claim 1, wherein (i) the mineral coating comprises a calcium-containing mineral, the calcium-containing mineral is at least one of apatite, hydroxyapatite, α-tricalcium phosphate, β-tricalcium phosphate, amorphous calcium phosphate, dicalcium phosphate, octacalcium phosphate, calcium carbonate, or a carbonated-substituted calcium-deficient hydroxyapatite, or anorganic bone; or (ii) the lyophilized porous macroparticles (14) comprise tricalcium phosphate and hydroxyapatite.

8. The bone implant (10) of claim 1, wherein the bone implant (10) is configured to be hydrated before, during or after the bone implant (10) is implanted into a bone defect with a fluid, comprising: bone marrow aspirate, saline, sterile water, blood for injection, phosphate buffered saline, dextrose, Ringer's lactated solution, or a combination thereof.

9. A method of making a bone implant (10), the method comprising providing a plurality of porous macroparticles (14) comprising ceramic material; coating the plurality of porous macroparticles (14) with a mineral coating comprising a plate-like nanostructure comprising nanoparticles having a size range from 10 to 100 nanometers; adding the plurality of coated porous macroparticles (14) to a collagen slurry to form a mixture; wherein the mineral coating of the porous macroparticles (14) comprises nanoparticles having a carbonate-substituted, calcium-deficient hydroxyapatite component; and lyophilizing the mixture comprising the macroparticles (14) to form the bone implant (10) comprising a plurality of recesses, projections or a combination thereof.

10. The method of claim 9, wherein the lyophilizing the mixture comprising the nanoparticles occurs in a mold to form square, cylindrical, corrugated, notches, curves, waffles, or hexagonal honeycomb shapes in a pattern or randomly about the bone implant (10).

11. The method of claim 9, wherein the plurality of recesses, projections or a combination thereof are etched into the implant.

12. The method of claim 9, wherein the plurality of recesses, projections or a combination thereof are radiographic markers, hydration channels, or indicia to determine bone growth within or bone growth adjacent to the bone implant (10).

13. The method of claim 9, wherein the plurality of porous particles comprises ceramic material in an amount from about 50 to about 98 wt. % and collagen in an amount from about 2 to about 50 wt. % based on a total weight of each of the lyophilized porous macroparticles (14), each of the lyophilized porous macroparticles (14) having an average diameter from about 0.1 mm to about 10 mm.

14. The method of claim 9, wherein the bone implant (10) has pores from about 50 microns to about 500 microns for bone growth.

15. The method of claim 9, wherein the bone implant (10) has pores from about 500 microns to about 5 mm for vascularization.

## Patentansprüche

1. Knochenimplantat (10), umfassend eine Vielzahl von lyophilisierten porösen Makropartikeln (14) aus Keramikmaterial und Kollagen, wobei die Vielzahl lyophilisierter poröser Makropartikel (14) mit einer Mineralbeschichtung überzogen ist, wobei die Mineralbeschichtung ein nanogroßes Merkmal umfasst, das eine carbonatsubstituierte, calciumarme Hydroxylapatitkomponente umfasst, wobei das Knochenimplantat (10) eine Vielzahl von Vertiefungen, Vorsprüngen oder eine Kombination davon umfasst,
wobei die Mineralbeschichtung eine plattenartige Nanostruktur umfasst, die Nanopartikel mit einer Größe im Bereich von 10 bis 100 Nanometern umfasst.

2. Knochenimplantat (10) nach Anspruch 1, wobei die Vertiefungen, Vorsprünge oder eine Kombination davon quadratische, zylindrische, gewellte, Kerben-, Kurven-, Waffel- oder sechseckige Wabenformen in einem Muster oder zufällig um das Knochenimplantat (10) herum bilden.

3. Knochenimplantat (10) nach Anspruch 2, wobei die Vielzahl von Vertiefungen, Vorsprüngen oder eine Kombination davon radiologische Markierungen, Hydratationskanäle oder Kennzeichen zum Bestimmen des Knochenwachstums in oder neben dem Knochenimplantat (10) sind.

4. Knochenimplantat (10) nach Anspruch 1, wobei die Vielzahl der lyophilisierten porösen Makropartikel (14) Keramikmaterial in einer Menge von etwa 50 bis etwa 98 Gew.-% und Kollagen in einer Menge von etwa 2 bis etwa 50 Gew.-%, bezogen auf das Gesamtgewicht jedes der lyophilisierten porösen Makropartikel (14), umfasst, wobei jedes der lyophilisierten porösen Makropartikel (14) einen durchschnittlichen Durchmesser von etwa 0,05 mm bis etwa 10 mm aufweist.

5. Knochenimplantat (10) nach Anspruch 1, wobei das Knochenimplantat (10) rollbar ist und (i) eine Dicke von etwa 1 mm bis etwa 4 mm aufweist und bis zu 83 % Keramikmaterial umfasst, und/oder (ii) eine Oberfläche von mindestens etwa 1 m ²/g bis etwa 100 m²/g.

6. Knochenimplantat (10) nach Anspruch 1, wobei das Knochenimplantat (10) Poren (i) von etwa 50 Mikrometern bis etwa 500 Mikrometern für das Knochenwachstum; oder (ii) von etwa 500 Mikrometern bis etwa 5 mm zur Vaskularisierung aufweist.

7. Knochenimplantat (10) nach Anspruch 1, wobei (i) die Mineralbeschichtung ein kalziumhaltiges Mineral umfasst, wobei das kalziumhaltige Mineral mindestens eines ist von Apatit, Hydroxylapatit, α-Trikalziumphosphat, β-Trikalziumphosphat, amorphem Kalziumphosphat, Dikalziumphosphat, Oktakalziumphosphat, Kalziumkarbonat oder einem karbonatsubstituierten kalziumarmen Hydroxylapatit oder anorganischem Knochen; oder (ii) die lyophilisierten porösen Makropartikel (14) Tricalciumphosphat und Hydroxylapatit umfassen.

8. Knochenimplantat (10) nach Anspruch 1, wobei das Knochenimplantat (10) konfiguriert ist, um vor, während oder nach der Implantation des Knochenimplantats (10) in einen Knochendefekt mit einem Fluid hydratisiert zu werden, umfassend: Knochenmarkaspirat, Kochsalzlösung, steriles Wasser, Blut für Injektionszwecke, phosphatgepufferte Kochsalzlösung, Dextrose, Ringer-Laktatlösung oder eine Kombination davon.

9. Verfahren zum Herstellen eines Knochenimplantats (10), das Verfahren umfassend Bereitstellen einer Vielzahl poröser Makropartikel (14) aus keramischem Material; Beschichten der Vielzahl poröser Makropartikel (14) mit einer Mineralbeschichtung, die eine plattenartige Nanostruktur umfasst, die Nanopartikel mit einer Größe im Bereich von 10 bis 100 Nanometern umfasst; Hinzufügen der Vielzahl beschichteter poröser Makropartikel (14) zu einer Kollagenaufschlämmung, um eine Mischung zu bilden; wobei die Mineralbeschichtung der porösen Makropartikel (14) Nanopartikel mit einer carbonatsubstituierten, calciumarmen Hydroxylapatitkomponente umfasst; und Lyophilisieren der Mischung, die die Makropartikel (14) umfasst, um das Knochenimplantat (10) zu bilden, das eine Vielzahl von Vertiefungen, Vorsprüngen oder eine Kombination davon umfasst.

10. Verfahren nach Anspruch 9, wobei das Lyophilisieren der Mischung, die die Nanopartikel umfasst, in einer Form erfolgt, um quadratische, zylindrische, gewellte, gekerbte, gebogene, waffelförmige oder sechseckige Wabenformen in einem Muster oder zufällig um das Knochenimplantat (10) herum auszubilden.

11. Verfahren nach Anspruch 9, wobei die Vielzahl von Vertiefungen, Vorsprüngen oder eine Kombination davon in das Implantat geätzt werden.

12. Verfahren nach Anspruch 9, wobei die Vielzahl von Vertiefungen, Vorsprüngen oder eine Kombination davon radiologische Markierungen, Hydratationskanäle oder Kennzeichen sind, um das Knochenwachstum in oder neben dem Knochenimplantat (10) festzustellen.

13. Verfahren nach Anspruch 9, wobei die Vielzahl der porösen Partikel Keramikmaterial in einer Menge von etwa 50 bis etwa 98 Gew.-% und Kollagen in einer Menge von etwa 2 bis etwa 50 Gew.-%, bezogen auf das Gesamtgewicht jedes der lyophilisierten porösen Makropartikel (14), umfasst, wobei jedes der lyophilisierten porösen Makropartikel (14) einen durchschnittlichen Durchmesser von etwa 0,1 mm bis etwa 10 mm aufweist.

14. Verfahren nach Anspruch 9, wobei das Knochenimplantat (10) Poren von etwa 50 Mikrometern bis etwa 500 Mikrometern für das Knochenwachstum aufweist.

15. Verfahren nach Anspruch 9, wobei das Knochenimplantat (10) Poren von etwa 500 Mikrometern bis etwa 5 mm zur Vaskularisierung aufweist.

## Revendications

1. Implant osseux (10) comprenant une pluralité de macroparticules poreuses lyophilisées (14) comprenant un matériau céramique et du collagène, la pluralité de macroparticules poreuses lyophilisées (14) recouvertes d'un revêtement minéral, le revêtement minéral comprenant une caractéristique de taille nanométrique comprenant un composant d'hydroxyapatite substitué au carbonate et déficient en calcium, l'implant osseux (10) comprenant une pluralité d'évidements, de saillies ou une combinaison de ceux-ci,
dans lequel le revêtement minéral comprend une nanostructure en forme de plaque comprenant des nanoparticules ayant une taille comprise entre 10 et 100 nanomètres.

2. Implant osseux (10) selon la revendication 1, dans lequel les évidements, les saillies ou une combinaison de ceux-ci forment des formes carrées, cylindriques, ondulées, des encoches, des courbes, des formes gaufrées, hexagonales en nid d'abeille dans un motif ou de façon aléatoire autour de l'implant osseux (10).

3. Implant osseux (10) selon la revendication 2, dans lequel la pluralité d'évidements, de saillies ou une combinaison de ceux-ci sont des marqueurs radiographiques, des canaux d'hydratation ou des indices pour déterminer la croissance osseuse à l'intérieur de l'implant osseux ou la croissance osseuse adjacente à l'implant osseux (10).

4. Implant osseux (10) selon la revendication 1, dans lequel la pluralité de macroparticules poreuses lyophilisées (14) comprend un matériau céramique dans une proportion d'environ 50 à environ 98 % en poids et du collagène dans une proportion d'environ 2 à environ 50 % en poids en fonction d'un poids total de chacune des macroparticules poreuses lyophilisées (14), chacune des macroparticules poreuses lyophilisées (14) ayant un diamètre moyen d'environ 0,05 mm à environ 10 mm.

5. Implant osseux (10) selon la revendication 1, dans lequel l'implant osseux (10) peut être enroulé et a (i) une épaisseur d'environ 1 mm à environ 4 mm et comprend jusqu'à 83 % de matériau céramique ; et/ou (ii) une surface d'au moins environ 1 m²/g à environ 100 m²/g.

6. Implant osseux (10) selon la revendication 1, dans lequel l'implant osseux (10) a des pores (i) d'environ 50 microns à environ 500 microns pour la croissance osseuse ; ou (ii) d'environ 500 microns à environ 5 mm pour la vascularisation.

7. Implant osseux (10) selon la revendication 1, dans lequel (i) le revêtement minéral comprend un minéral contenant du calcium, le minéral contenant du calcium est au moins l'un parmi l'apatite, l'hydroxyapatite, le phosphate α-tricalcique, le phosphate β-tricalcique, le phosphate de calcium amorphe, le phosphate dicalcique, le phosphate octacalcique, le carbonate de calcium, ou une hydroxyapatite carbonatée-substituée déficiente en calcium, ou l'os anorganique ; ou (ii) les macroparticules poreuses lyophilisées (14) comprennent du phosphate tricalcique et de l'hydroxyapatite.

8. Implant osseux (10) selon la revendication 1, dans lequel l'implant osseux (10) est conçu pour être hydraté avant, pendant ou après l'implantation de l'implant osseux (10) dans un défaut osseux avec un fluide, comprenant : de l'aspirat de moelle osseuse, une solution saline, de l'eau stérile, du sang pour injection, une solution saline tamponnée au phosphate, du dextrose, de la solution lactée de Ringer, ou une combinaison de ceux-ci.

9. Procédé de fabrication d'un implant osseux (10), le procédé comprenant la fourniture d'une pluralité de macroparticules poreuses (14) comprenant du matériau céramique ; le revêtement de la pluralité de macroparticules poreuses (14) avec un revêtement minéral comprenant une nanostructure en forme de plaque comprenant des nanoparticules ayant une taille comprise entre 10 et 100 nanomètres ; l'ajout de la pluralité de macroparticules poreuses revêtues (14) à une suspension de collagène pour former un mélange ; dans lequel le revêtement minéral des macroparticules poreuses (14) comprend des nanoparticules ayant un composant d'hydroxyapatite substitué au carbonate et déficient en calcium ; et la lyophilisation du mélange comprenant les macroparticules (14) pour former l'implant osseux (10) comprenant une pluralité d'évidements, de saillies ou une combinaison de ceux-ci.

10. Procédé selon la revendication 9, dans lequel la lyophilisation du mélange comprenant les nanoparticules se produit dans un moule pour former des formes carrées, cylindriques, ondulées, des encoches, des courbes, des formes gaufrées, hexagonales en nid d'abeille dans un motif ou de façon aléatoire autour de l'implant osseux (10).

11. Procédé selon la revendication 9, dans lequel la pluralité d'évidements, de saillies ou une combinaison de ceux-ci sont gravés dans l'implant.

12. Procédé selon la revendication 9, dans lequel la pluralité d'évidements, de saillies ou une combinaison de ceux-ci sont des marqueurs radiographiques, des canaux d'hydratation ou des indices pour déterminer la croissance osseuse à l'intérieur de l'implant osseux ou la croissance osseuse adjacente à l'implant osseux (10).

13. Procédé selon la revendication 9, dans lequel la pluralité de particules poreuses comprend un matériau céramique dans une proportion d'environ 50 à environ 98 % en poids et du collagène dans une proportion d'environ 2 à environ 50 % en poids en fonction d'un poids total de chacune des macroparticules poreuses lyophilisées (14), chacune des macroparticules poreuses lyophilisées (14) ayant un diamètre moyen d'environ 0,1 mm à environ 10 mm.

14. Procédé selon la revendication 9, dans lequel l'implant osseux (10) a des pores d'environ 50 microns à environ 500 microns pour la croissance osseuse.

15. Procédé selon la revendication 9, dans lequel l'implant osseux (10) a des pores d'environ 500 microns à environ 5 mm pour la vascularisation.
